# EUROPEAN PATENT APPLICATION

(11) **EP 3 098 691 A1**
(43) Date of publication of application: **30.11.2016**
(21) Application number: 15169960.0
(22) Date of filing: 29.05.2015
(51) Int. Cl.: G06F 3/01, A61B 5/107

(54) **FLEX SENSOR AND INSTRUMENTED GLOVE**

(71) Applicant: Manus Machinae B.V., 5617 BC Eindhoven (NL)
(72) Inventor: Stumpel, Stijn, 5652 EH Eindhoven (NL); Witteveen, Maarten, 1033 CP Amsterdam (NL); Van Veenendaal, Tim, 5672 SP Nuenen (NL); Van den Brink, Stephan, 5613 KS Eindhoven (NL)
(74) Representative: Nederlandsch Octrooibureau

(57) **Abstract**

The invention relates to a flex sensor, and more particularly, to an instrumented glove comprising one or more such flex sensors. A flex sensor comprising an elongated body of a material whose electrical resistance depends on a magnitude of a bending of the elongated body. At least three electrically conducting lines are attached to the elongated body. Measuring the resistance between the three electrically conducting lines gives the magnitude of the bending of the elongated body. The invention further relates to an assembly of flex sensors and to a data glove comprising at least one flex sensor.

## Description

### Field of the invention

The invention relates to a flex sensor, and more particularly, to an instrumented glove comprising one or more flex sensors.

### Background of the invention

The increased demand for computer games, 3D media, and user-intuitive controller emphasizes the need for a data glove. A data glove is generally a glove that fits over at least a part of a user's hand and detects movement of the user's fingers and/or hand. Data gloves (hereafter also called "instrumented gloves") are commonly used for controlling computer games, video games, motion capturing, and in robotics. For example, motion capturing is used in the entertainment industry when the motions of a number of points on a hand are recorded in a computer, and the recorded motions then transferred to an animated hand in order to impart a greater sense of reality to the animation.

Data gloves have been implemented using several different approaches, including strain gauges attached to the glove to detect a magnitude of a bending of a finger.

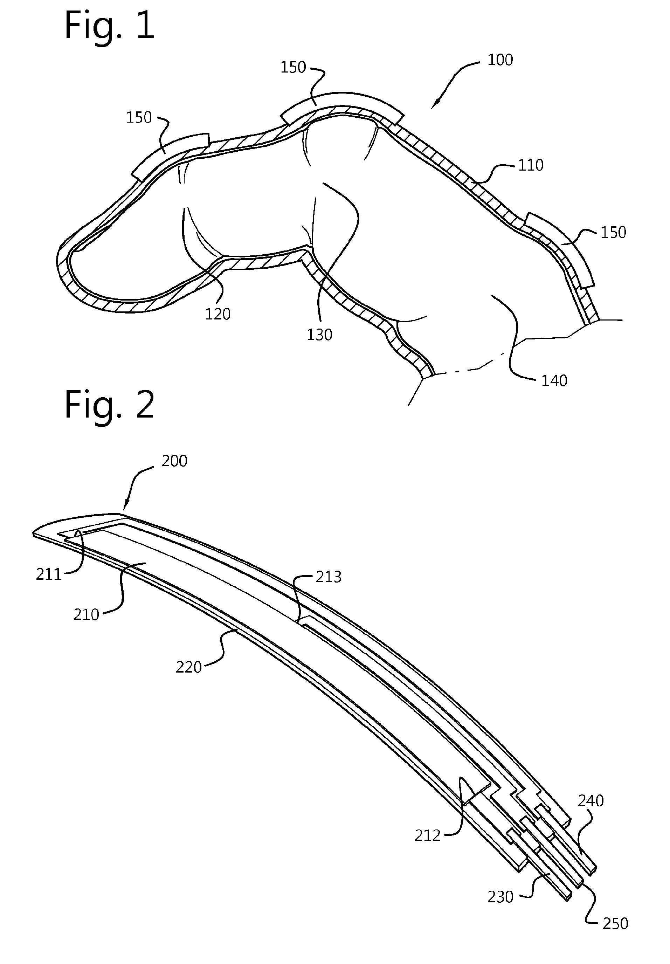

Figure 1 is a sectional view of one finger 100 of a conventional instrumented glove with the finger 100 having a bent orientation. The instrumented glove comprises of parts 110 covering at least parts of five fingers of a user's hand: the thumb, the index finger, the middle finger, the ring finger, and the small finger. The parts 110 can be flexible. Each of the index finger, the middle finger, the ring finger, and the small finger has a distal interphalangeal, DIP, joint 120, a proximal interphalangeal, PIP, joint 130, and a metacarpophalangeal, MCP joint 140. The thumb has a interphalangeal, IP, joint, and a MCP joint. Furthermore, the user's hand has five carpometacarpal, CMC, joints connecting the wrist.

The parts 110 covering the thumb, the index finger, the middle finger, the ring finger, and the small finger comprise of flexible materials. Straing gauges 150 are embedded in the parts 110 in the vicinity of the DIP 120, PIP 130, MCP 140, and IP joints of the fingers.

By bending the fingers, signals are generated as electrical current through the strain gauge elements modulated by the strain imposed when the fingers are bent. Each strain gauge element detects one of the DIP 120, PIP 130, IP, MCP 140 joints. The signals from the strain gauge elements are processed by a processor (not shown).

However, in order to detect the bending of multiple joints of the finger, multiple strain gauges are needed per finger. The strain gauges can be complex. Consequently, the fabrication of the strain gauges is expensive and the cost of the data glove is thereby increased.

There is therefore a need to produce sensors suitable for a data glove where the number of sensors detecting the bending of the finger's joints can be reduced, and at the same time maintaining the ability of detecting the bending of the finger's joints.

### Summary of the invention

An object of the present invention is to provide an improved flex sensor.

A further object of the present invention is to provide an improved instrumented glove comprising at least one flex sensor.

The invention provides a flex sensor comprising an elongated body of a material whose electrical resistance depends on a magnitude of a bending of the elongated body; at least three electrically conducting lines attached to the elongated body; wherein a first electrical conducting line is attached to a first part of the elongated body; a second electrical conducting line is attached to a second part of the elongated body; a third electrical conducting line is attached to a third part of the elongated body between the first and the second parts of the elongated body.

Advantageously, by measuring the electrical resistance between the first and the third part and by measuring the electrical resistance between the second and the third part, a single flex sensor can be used to generate two different bending states. The invention is not limited to the embodiment with three electrically conducting lines attached to three parts of the elongated body. Also an embodiment with four conducting lines is possible, with the fourth conducting line attached to a fourth part of the elongated body between the first and the third part or between the second and third part. In principle, any integer number of conducting lines can be used.

The invention also provides an assembly of a flexible and/or stretchable substrate and a plurality of flex sensors as described above, wherein each of the plurality of flex sensors is provided on the flexible and/or stretchable substrate.

The invention also provides an instrumented glove comprising at least one flex sensor as described above, or the assembly of the flexible and/or stretchable substrate and the plurality of flex sensors described above.

Further embodiments are disclosed in the attached claims.

### Brief description of the Figures

Embodiments of the present invention will be described hereinafter, by way of example only, with reference to the accompanying drawings which are schematic in nature and therefore not necessarily drawn to scale. Furthermore, like reference signs in the drawings relate to like elements.
Figure 1 is a sectional view of one finger of a conventional instrumented glove with the finger having a bent orientation.
Figure 2 schematically shows a flex sensor according to embodiments of the present disclosure.
Figure 3 schematically shows a flex sensor according to embodiments of the present disclosure.
Figure 4 is a sectional view of one finger of an instrumented glove with a flex sensor incorporated according to embodiments of the present disclosure.
Figure 5 schematically shows a back view of an instrumented glove according to embodiments of the present disclosure.
Figure 6 schematically shows an assembly of a flexible and/or stretchable substrate and a plurality of flex sensors according to embodiments of the present disclosure.
Figure 7 schematically shows an assembly of a stretchable substrate and a plurality of flex sensors according to embodiments of the present disclosure.
Figure 8 schematically shows a back view of an instrumented glove according to other embodiments of the present disclosure.
Figure 9 schematically shows a back view of an instrumented glove according to other embodiments of the present disclosure.
Figure 10 schematically shows a flex sensor incorporated in an instrumented glove in areas of a palm.
Figure 11 schematically shows a back view of an instrumented glove according to other embodiments of the present disclosure.

### Detailed description

Figure 2 schematically shows a flex sensor 200 according to embodiments of the present disclosure. The flex sensor 200 comprises of a polymer ink part 210 that has conductive particles embedded in it. The conductive particles give the polymer ink part 210 an electrical resistance. The polymer ink part 210 may be provided on a flexible and/or stretchable substrate 220, and has an elongated shape. When the flex sensor 200 is straight (not bent), the polymer ink part 210 has an original electrical resistance value. When the flex sensor 200 is bent, the electrical resistance value of the polymer ink part 210 deviates from the original electrical resistance value. The electrical resistance value of the polymer ink part 210 scales in a polynomial way with a magnitude of the bending of the flex sensor 200. Bending of the flex sensor 200 on opposite sides of an axis of movement can result in an asymmetry of the electrical resistance value.

In order to measure the electrical resistance value of the polymer ink part 210, the polymer ink part 210 is electrically contacted by at least three electrical conducting lines. A first electrical conducting line 230 contacts a first part 211 of the polymer ink part 210, a second electrical conducting line 240 contacts a second part 212 of the polymer ink part 210, and a third electrical conducting line 250 contacts a third part 213 of the polymer ink part 210 between the first 211 and the second 212 parts of the polymer ink part 210. The first part 211 and the second part 212 may be located at the opposing distal ends of the polymer ink part 210. The third part 213 may be located in the middle of the polymer ink part 210, or it may be closer to either the first 211 or the second part 212. The first, the second, and the third electrical conducting lines 230, 240, 250 may be embedded in the flexible and/or stretchable substrate 220.

By measuring the resistance between the first electrical conducting line 230 and the third electrical conducting line 250, the magnitude of the bending of the polymer ink part between the first electrical conducting line 230 and the third electrical conducting line 250 can be determined. By measuring the resistance between the second electrical conducting line 250 and the third electrical conducting line 250, the magnitude of the bending of the polymer ink part between the second electrical conducting line 240 and the third electrical conducting line 250 can be determined. By measuring the resistance between the first electrical conducting line 230 and the second electrical conducting line 240, and floating the third electrical conducting line 250, the magnitude of the bending of the polymer ink part between the first electrical conducting line 230 and the second electrical conducting line 240 can be determined. Therefore, by measuring a different combination of the electrical conducting lines, the magnitude of the bending at different spatial locations can be distinguished. Additional electrical conducting lines can electrically contact the polymer ink part 210 between the two ends, such that the magnitude of the bending at more than two spatial locations can be distinguished.

The above described electrical resistance values can be measured by a processing unit (not shown). The processing unit may comprise resistance detection means. The processing unit may comprise a look-up table (LUT) or calculation means to convert a resistance measurement to a bending state value (e.g. an angle of bending of the part of the sensor corresponding to the measured resistance). The processing unit can be integrated in or near the flex sensor 200. A flex sensor unit can be provided as an integrated package of a (flexible and/or stretchable) substrate (not shown), the one or more flex sensors 200, and the processing unit connected to the one or more flex sensors 200. The processing unit may be integrated with a single flex sensor. The (flexible and/or stretchable) substrate and the flexible and/or stretchable substrate 220 may comprise the same material.

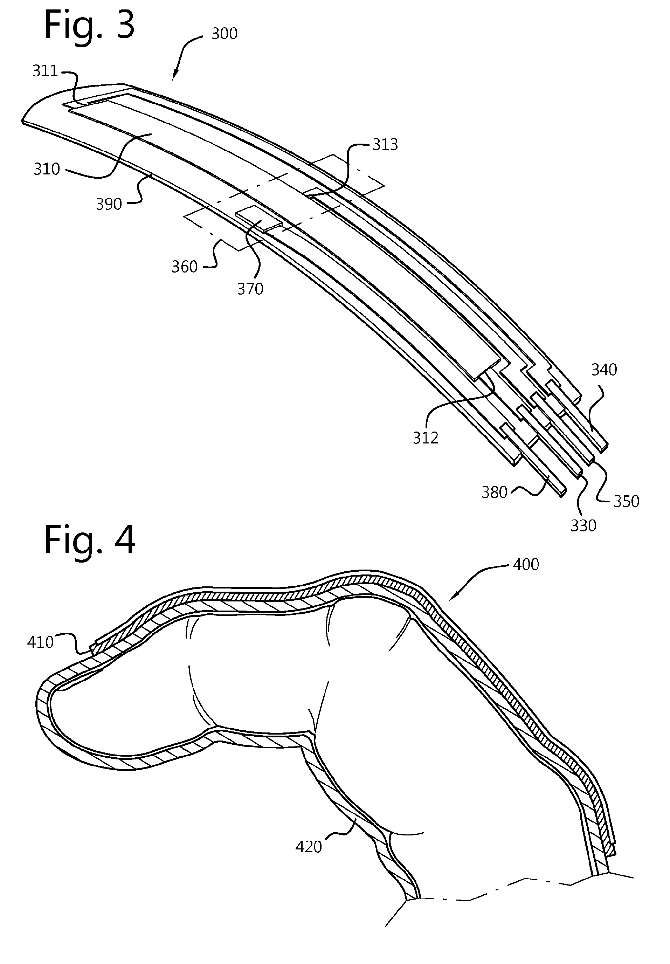

Figure 3 schematically shows a flex sensor 300 according to embodiments of the present disclosure. The flex sensor 300 comprises of a polymer ink part 310 and has an elongated shape. The polymer ink part 310 is electrically contacted by at least three electrical conducting lines. A first electrical conducting line 330 contacts a first part 311 of the polymer ink part 310, a second electrical conducting line 340 contacts a second part 312 of the polymer ink part 310, and a third electrical conducting line 350 contacts a third part 313 of the polymer ink part 310 between the first and the second parts of the polymer ink part 310. The first part 311 and the second part 312 may be located at the opposing distal ends of the polymer ink part 310. A section 360 of the polymer ink part 310 and one or more of the electrical conducting lines 330, 340, 350 is provided on or in a rigid substrate, and an integrated circuit 370 is provided on or in the rigid substrate. The integrated circuit 370 may be an inertia measurement unit. The inertia measurement unit may have one or more electrical conducting lines 380 contacted to the inertia measurement unit. The flex sensor 300 excluding the rigid section 360 may be provided on or in a flexible and/or stretchable substrate 390.

Figure 4 is a sectional view of one finger 400 of an instrumented glove with a flex sensor 410 incorporated according to embodiments of the present disclosure. The flex sensor 410 may be the same as the flex sensor 200 or the flex sensor 300. The finger 400 of the instrumented glove comprises a part 420 covering at least parts of one of the index finger, the middle finger, the ring finger, and the small finger. However, the principle also applies to the thumb. The flex sensor 410 is embedded in the back of the finger 400 of the instrumented glove, covering the DIP joint 110, the PIP joint 120, and the MCP joint 130. The middle point of the polymer ink part 210 where the third electrical conducting line 250 is contacted is situated between the PIP joint 120 and the MCP joint 130. Such that the flex sensor 410 can detect both the bending of the MCP joint, and the combined bending of the DIP and the PIP joints. For the flex sensor 410 covering the IP joint and the MCP joint of the thumb, the middle point of the polymer ink part 210 where the third electrical conducting line 250 is contacted is situated between the IP joint and the MCP joint. The rigid section 360 of the flex sensor 300 comprising the inertia measurement unit may be situated between the IP joint and the MCP joint of the thumb.

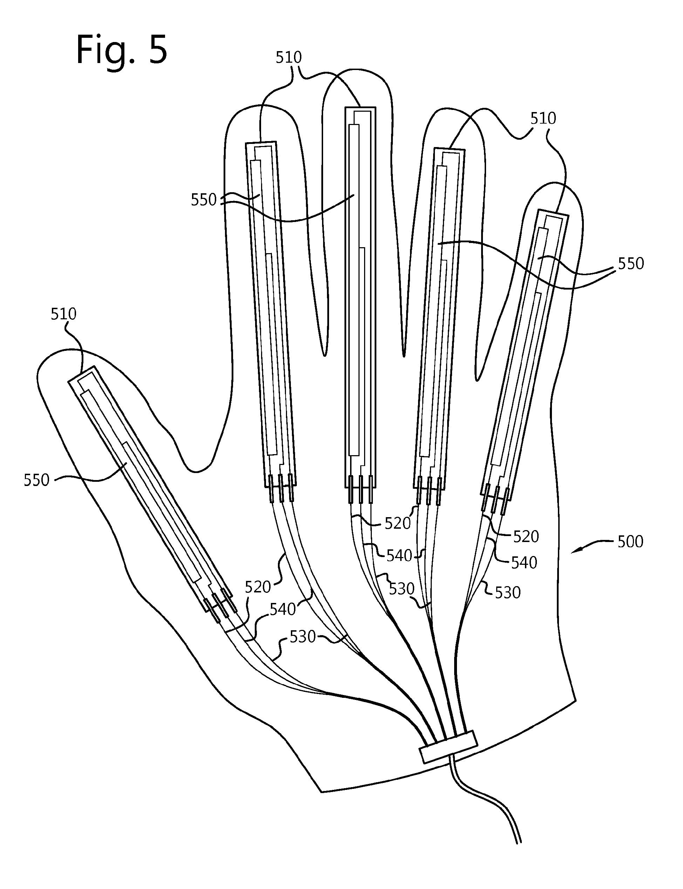

Figure 5 schematically shows a back view of an instrumented glove 500 according to embodiments of the present disclosure. A flex sensor 510 is embedded in each finger of the instrumented glove 500. The flex sensor 510 may be the same as the flex sensor 200 or the flex sensor 300. A plurality of cables connecting a first electrical conducting line 520 (contacting a first part of a polymer ink part 550), a second electrical conducting line 530 (contacting a second part of the polymer ink part 550), and a third electrical conducting line 540 (contacting a third part of the polymer ink part 550 between the two parts of the polymer ink part 550) of the flex sensor 410 may come together at one place on the back of the hand. The cables connecting the third electrical conducting line 540 of each flex sensor 510 may be connected to each other in order to reduce the number of cables. The flex sensors 510 may be placed at a back side of the instrumented glove 500 and/or at a front side of the instrumented glove 500. The flex sensor 510 embedded in the thumb of the instrumented glove 500 may be the same as the flex sensor 300, including the inertia measurement unit 370 provided on a rigid substrate (not shown).

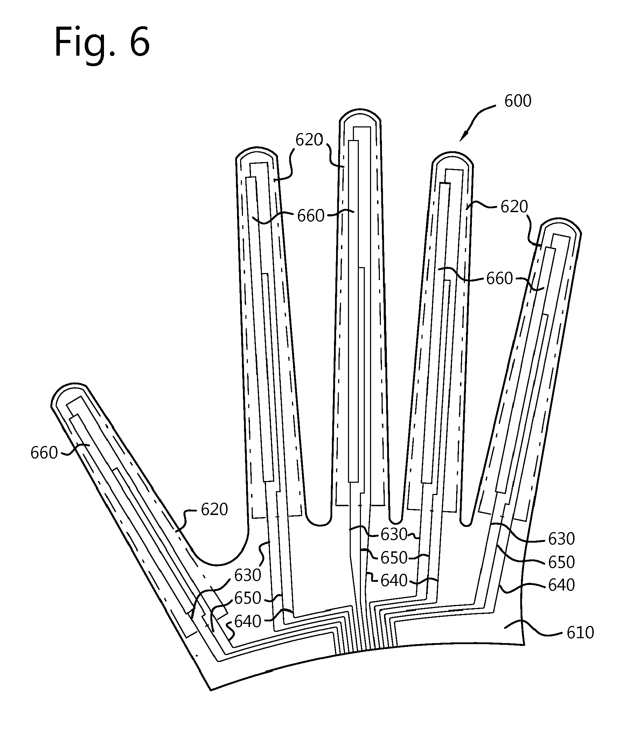

Figure 6 schematically shows an assembly of a flexible and/or stretchable substrate 610 and a plurality of flex sensors 620 wherein each of the plurality of flex sensors 620 is provided on the flexible and/or stretchable substrate 610. The flexible and/or stretchable substrate 610 may cover more than one finger, and may have at least a part of a shape of the hand. The flex sensor 620 may be the same as the flex sensor 200 or the flex sensor 300. A plurality of cables connecting a first electrical conducting line 630 (contacting a first part of a polymer ink part 660), a second electrical conducting line 640 (contacting a second part of the polymer ink part 660), and a third electrical conducting line 650 (contacting a third part of the polymer ink part 660 between the two parts of the polymer ink part 660) of the flex sensor 620 may come together at one place on or embedded in the flexible and/or stretchable substrate 610. The cables connecting the third electrical conducting line 650 of each flex sensor 620 may be connected to each other in order to reduce the number of cables. The flex sensor 620 embedded in the thumb of the instrumented glove 600 may be the same as the flex sensor 300, including the inertia measurement unit 370 provided on a rigid substrate (not shown).

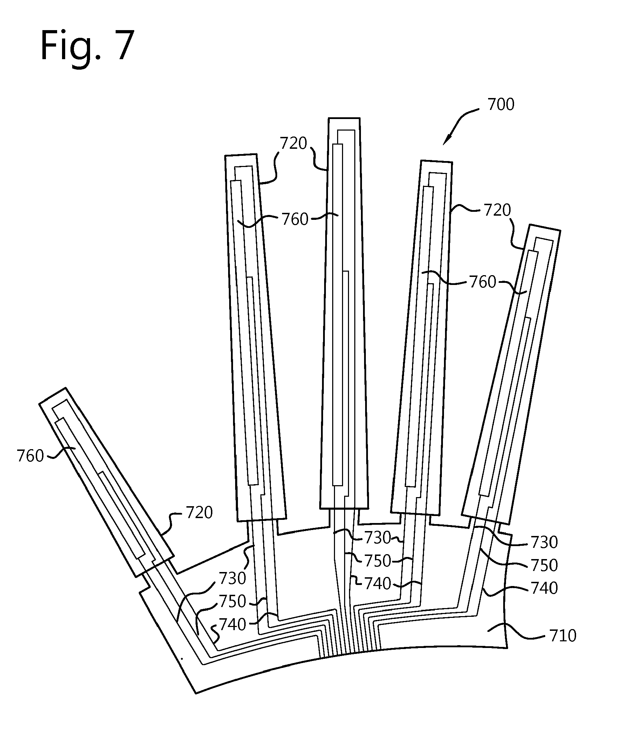

Figure 7 schematically shows an assembly of a stretchable part 710 and a plurality of flex sensors 720 wherein each of the plurality of flex sensors 720 is connected to the stretchable part 710. The stretchable part 710 may be based on a stretchable circuit board technology. The assembly may cover more than one finger, and may have at least a part of a shape of the hand. The flex sensor 720 may be the same as the flex sensor 200 or the flex sensor 300. A plurality of cables connecting a first electrical conducting line 730 (contacting a first part of a polymer ink part 760), a second electrical conducting line 740 (contacting a second part of the polymer ink part 760), and a third electrical conducting line 750 (contacting a third part of the polymer ink part 760 between the two parts of the polymer ink part 760) of the flex sensor 720 may come together at one place on or imbedded in the stretchable part 710. The cables connecting the third electrical conducting line 750 of each flex sensor 720 may be connected to each other in order to reduce the number of cables. The flex sensor 720 embedded in the thumb of the instrumented glove 700 may be the same as the flex sensor 300, including the inertia measurement unit 370 provided on a rigid substrate (not shown).

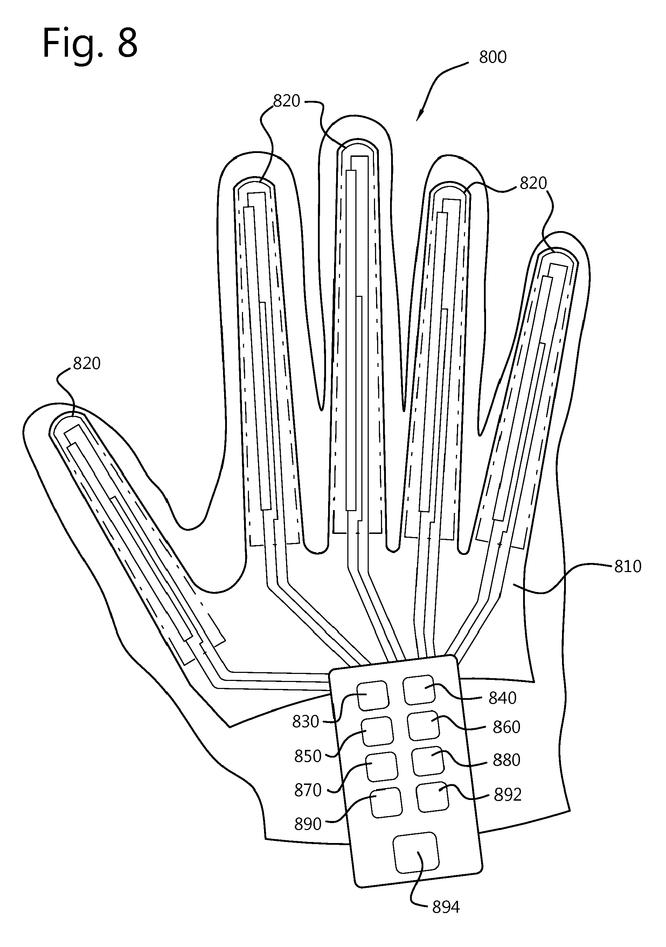

Figure 8 schematically shows a back view of an instrumented glove 800 according to embodiments of the present disclosure. The instrumented glove 800 comprises an assembly of a flexible and/or stretchable substrate 810 and a plurality of flex sensors 820 wherein each of the plurality of flex sensors 820 is provided on the flexible and/or stretchable substrate 810. The flex sensor 820 may be the same as the flex sensor 200 or the flex sensor 300. The instrumented glove 800 may include one or more of an accelerometer module 830 to measure an acceleration of the hand and/or the fingers, a gyroscope module 840 to measure tilting angles of the hand and/or the fingers, a magnetometer module 850, a hall-effect flux producing element 860, a hall-effect sensor 870, a wireless communication module 880, and a battery module 890. The wireless communication module 880 can, for example be a Bluetooth module. The instrumented glove may include a rumbler module 892, which can be a vibration device, a piezoelectric film, or a linear vibrator. The rumbler module 892 may be incorporated in the instrumented glove 800 in areas of the fingers, and/or the back of the hand, and/or the palm of the hand. Via the rumble module 820, the user receives feedbacks of interactions that are carried out by the user. The flex sensor 820 embedded in the thumb of the instrumented glove 800 may be the same as the flex sensor 300, including the inertia measurement unit 370 provided on a rigid substrate (not shown).

The instrumented glove 800 may include a processing module 894 that measures electrical resistance values of the flex sensors 820. The processing module 894 may comprise resistance detection means. The processing module 894 may comprise a look-up table (LUT) or calculation means to convert a resistance measurement to a bending state value (e.g. an angle of bending of the part of the sensor corresponding to the measured resistance). The processing module 894 can be integrated in or near the flex sensor 820.

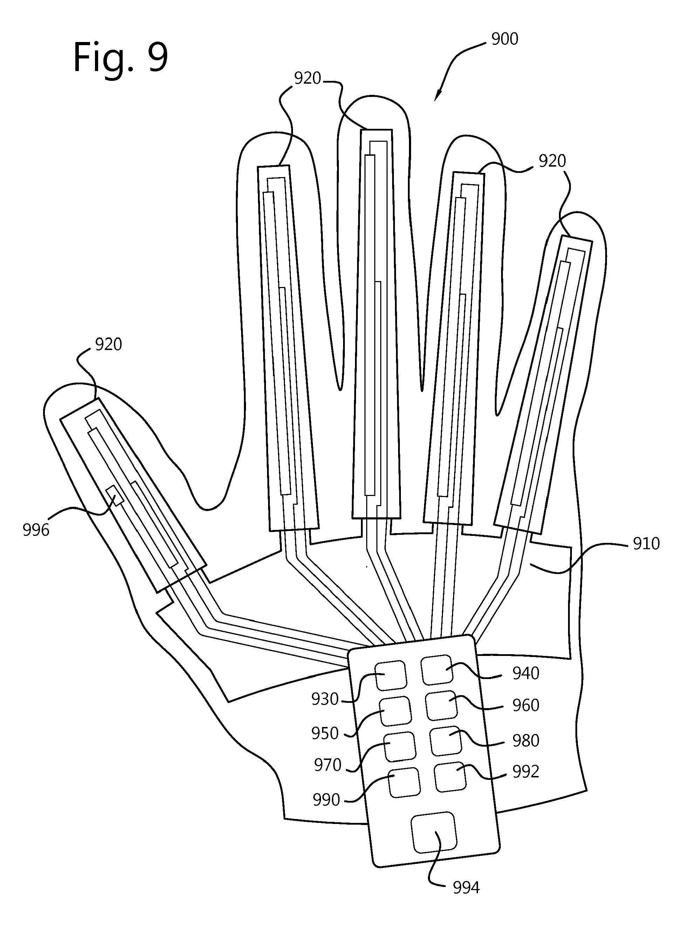

Figure 9 schematically shows a back view of an instrumented glove 900 according to embodiments of the present disclosure. The instrumented glove 900 comprises an assembly of a stretchable part 910 and a plurality of flex sensors 920 wherein each of the plurality of flex sensors 920 is connected to the stretchable part 910. The stretchable part 910 may be based on a stretchable circuit board technology. The flex sensor 920 may be the same as the flex sensor 200 or the flex sensor 300. The instrumented glove 900 may include one or more of an accelerometer module 930 to measure an acceleration of the hand and/or the fingers, a gyroscope module 940 to measure tilting angles of the hand and/or the fingers, a magnetometer module 950, a hall-effect flux producing element 960, a hall-effect sensor 970, a wireless communication module 980, and a battery module 990. The wireless communication module 980 can, for example be a Bluetooth module. The instrumented glove may include a rumbler module 992, which can be a vibration device, a piezoelectric film, or a linear vibrator. The rumbler module 992 may be incorporated in the instrumented glove 900 in areas of the fingers, and/or the back of the hand, and/or the palm of the hand. Via the rumble module 920, the user receives feedbacks of interactions that are carried out by the user. The flex sensor 920 embedded in the thumb of the instrumented glove 900 may be the same as the flex sensor 300, including an inertia measurement unit 996 provided on a rigid substrate. Alternatively, the inertia measurement unite 996 maybe also be provided on a rigid substrate embedded in one or more of the other fingers.

The instrumented glove 900 may include a processing module 994 that measures electrical resistance values of the flex sensors 920. The processing module 994 may comprise resistance detection means. The processing module 994 may comprise a look-up table (LUT) or calculation means to convert a resistance measurement to a bending state value (e.g. an angle of bending of the part of the sensor corresponding to the measured resistance). The processing module 994 can be integrated in or near the flex sensor 920.

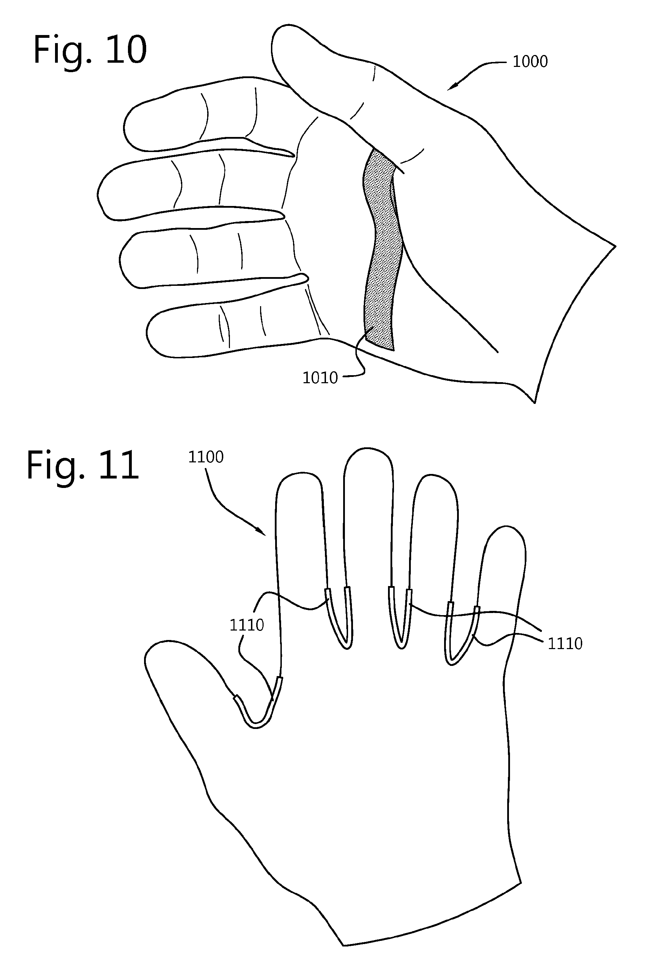

Figure 10 schematically shows a flex sensor 1010 incorporated in an instrumented glove 1000 in areas of the palm to sense hand motions, for example, contraction of the palm. A plurality of flex sensors 1010 may be incorporated in the instrumented glove 1000 in areas of the palm to increase the accuracy and/or precession of the sensing of the hand motion. The flex sensor 1010 may be the same as the flex sensor 200 or the flex sensor 300. Alternatively the flex sensors 1010 may be incorporated in the instrumented glove in areas of the back of the hand.

Figure 11 schematically shows a back view of an instrumented glove 1100 with flex sensors 1110 situated between the fingers. The flex sensor 1100 may be the same as the flex sensor 200 or the flex sensor 300. The flex sensors 1110 may detect a magnitude of a spreading of the fingers.

In the foregoing description of the figures, the invention has been described with reference to specific embodiments thereof. It will, however, be evident that various modifications and changes may be made thereto without departing from the scope of the invention as summarized in the attached claims.

In addition, many modifications may be made to adapt a particular situation or material to the teachings of the invention without departing from the essential scope thereof. Therefore, it is intended that the invention not be limited to the particular embodiments disclosed, but that the invention will include all embodiments falling within the scope of the appended claims.

In particular, combinations of specific features of various aspects of the invention may be made. An aspect of the invention may be further advantageously enhanced by adding a feature that was described in relation to another aspect of the invention.

It is to be understood that the invention is limited by the annexed claims and its technical equivalents only. In this document and in its claims, the verb "to comprise" and its conjugations are used in their non-limiting sense to mean that items following the word are included, without excluding items not specifically mentioned. In addition, reference to an element by the indefinite article "a" or "an" does not exclude the possibility that more than one of the element is present, unless the context clearly requires that there be one and only one of the elements. The indefinite article "a" or "an" thus usually means "at least one".

Although exemplary embodiments only detect the movements of the abovementioned joints, according to the invention more sensors can be applied to detect the motion of other joints.

## Claims

1. A flex sensor comprising
an elongated body (210) of a material whose electrical resistance depends on a magnitude of a bending of the elongated body;
at least three electrically conducting lines attached to the elongated body; wherein a first electrical conducting line (230) is attached to a first part (211) of the elongated body;
a second electrical conducting line (240) is attached to a second part (212) of the elongated body;
a third electrical conducting line (250) is attached to a third part (213) of the elongated body between the first part and the second part of the elongated body.

2. The flex sensor according to claim 1 further comprising a processing module for determining
a first bending state value based on a measured electrical resistance value between the first part of the elongated body and the third part of the elongated body; and
a second bending state value based on a measured electrical resistance value between the second part of the elongated body and the third part of the elongated body.

3. The flex sensor according to claim 1 or 2 wherein the material is or comprises a polymer ink.

4. The flex sensor according to any one of the preceding claims wherein the elongated body and the conducting lines (230, 240, 250) are provided on or in a flexible and/or stretchable substrate (220).

5. The flex sensor according to any one of the preceding claims wherein a section (360) of the elongated body is provided on or in a rigid substrate, and an integrated circuit (370) is provided on or in the rigid substrate.

6. The flex sensor according to claim 5 wherein the integrated circuit (370) is an inertia measurement unit.

7. An assembly of a flexible and/or stretchable substrate (610) and a plurality of flex sensors according to any one of claims 1-6 wherein each of the plurality of flex sensors is provided on the flexible and/or stretchable substrate.

8. The assembly according to claim 7 wherein the flexible and/or stretchable substrate (610) is shaped like at least a part of a hand.

9. The assembly according to claim 7 or 8 wherein at least four flex sensors are provided, each respective flex sensor arranged for sensing a bending state of a respective digit of a hand.

10. An assembly of a stretchable part (710) and a plurality of flex sensors according to any one of claims 1-6 wherein each of the plurality of flex sensors is connected to the stretchable part (710).

11. An instrumented glove comprising
at least one flex sensor (510) according to any one of claims 1-6,
or the assembly of the flexible and/or stretchable substrate (810) and the plurality of flex sensors (820) according to any one of the claims 7-9,
or the assembly of the stretchable part (910) and the plurality of flex sensors (920) according to claim 10.

12. The instrumented glove according to claim 11, wherein each flex sensor is configured to detect a bending state of a distal interphalangeal joint (120), or a proximal interphalangeal joint (130), or a interphalangeal joint, or a metacarpophalangeal joint (140), or a carpometacarpal joint, or any combination of the said joints.

13. The instrumented glove according to any one of claims 11-12, wherein the glove comprises a processor module (994) configured to detect a bending state of at least one of the flex sensors.

14. The instrumented glove according to any one of claims 11-13, wherein the glove comprises one or more of an accelerometer module (930), a gyroscope module (940), a magnetometer module (950), a wireless communication module (980), a hall-effect flux producing element (960), a hall-effect sensor (970), and a battery module (990).

15. The instrumented glove according to any one of claims 11-14, wherein the instrumented glove comprises a rumbler module (992), which comprises a vibration device, a piezoelectric film, or a linear vibrator.
